# EUROPÄISCHE PATENTSCHRIFT

(11) **EP 1 071 807 B1**
(45) Veröffentlichungstag und Bekanntmachung des Hinweises auf die Patenterteilung: **14.07.2010**
(21) Anmeldenummer: 99910248.6
(22) Anmeldetag: 20.02.1999
(51) Int. Cl.: C12P 41/00

(54) **VERFAHREN ZUR ENZYMATISCHEN ENANTIOMEREN-TRENNUNG VON 3(R)- UND 3(S)-HYDROXY-1- METHYL-4-(2,4, 6-TRIMETHOXYPHENYL)-1, 2,3,6- TETRAHYDRO-PYRIDIN BZW. DER CARBONSÄUREESTER**
METHOD FOR ENZYMATIC ENANTIOMER-SEPARATION OF 3(R)- AND 3(S)-HYDROXY-1- METHYL-4-(2,4, 6-TRIMETHOXYPHENYL)-1, 2,3,6- TETRAHYDRO-PYRIDINE OR ITS CARBOXYLIC ACID ESTERS
PROCEDE DE SEPARATION ENANTIOMERE ENZYMATIQUE DE 3(R)- ET DE 3(S)-HYDROXY-1- METHYL-4-(2,4, 6-TRIMETHOXYPHENYLE)-1, 2,3,6- TETRAHYDRO-PYRIDINE OU DE SES ESTERS D'ACIDE CARBOXYLIQUE

(30) Priorität: 06.03.1998 DE 19809649
(43) Veröffentlichungstag der Anmeldung: 31.01.2001
(73) Patentinhaber: Sanofi-Aventis Deutschland GmbH, 65929 Frankfurt am Main (DE)
(72) Erfinder: HOLLA, Wolfgang, D-65779 Kelkheim (DE)
(86) Internationale Anmeldenummer: PCT/EP1999/001113
(87) Internationale Veröffentlichungsnummer: WO 1999/045133

(56) Entgegenhaltungen:
- EP-A- 0 321 918
- EP-A- 0 474 129
- EP-A- 0 507 278
- US-A- 4 971 909
- DATABASE WPI Section Ch, Week 9418 Derwent Publications Ltd., London, GB; Class B03, AN 94-147005 XP002105673 & JP 06 090790 A (MERCIAN CORP) , 5. April 1994

## Beschreibung

Verfahren zur enzymatischen Enantiomeren-Trennung von 3(R)- und 3(S)-Hydroxy-1-methyl-4-(2,4,6-trimethoxyphenyl)-1,2,3,6-tetrahydro-pyridin bzw. der Carbonsäureester

Die Erfindung betrifft ein Verfahren zur Herstellung optisch reiner Verbindungen der Formel (I) durch stereodifferenzierende Umsetzung der Enantiomeren-Gemische mit Hilfe eines Enzyms.

3(S)- bzw. 3(R)- Hydroxy-1-methyl-4-(2,4,6-trimethoxyphenyl)-1,2,3,6-tetrahydropyridin (Verbindungen der Formel (I) mit R = H) bzw. deren Esterderivate (Verbindungen der Formel (I) mit R = COR¹) sind zentrale Bausteine bzw. Vorstufen der in der Patentanmeldung HMR 98/L 001 ("Verfahren zur Herstellung von (-)cis -3-Hydroxy-1-methyl-4(R)-(2,4,6-trimethoxyphenyl)piperidin") beschriebenen Synthese des Flavopiridols (HMR 1275 oder L 86 8275), des ersten potenten Inhibitors der cyclin-abhängigen Protein-Kinase (siehe z.B. Sedlacek, Hans Harald; Czech, Joerg; Naik, Ramachandra; Kaur, Gurmeet; Worland, Peter; Losiewicz, Michael; Parker, Bernard; Carlson, Bradley; Smith, Adaline; et al. Flavopiridol (L 86 8275; NSC 649890), a new kinase inhibitor for tumor therapy. Int. J. Oncol. (1996), 9(6), 1143-1168 oder Czech, Joerg; Hoffmann, Dieter; Naik, Ramachandra; Sedlacek, Hans-Harald; Antitumoral activity of flavone L 86 8275. Int. J. Oncol. (1995), 6(1), 31-36).

EP-A-0474 beschreibt dass Pyridinester werden mittels Enzym (z.B. Lipase) einer asymmetrischen Hydrolyse unteruzogen werden um die optischen Enantiomeren zu gewinnen.

Eine Racematspaltung bzw. Enantiomeren-Trennung der Verbindungen der Formel (I) ist nicht bekannt.

Es wurde nun gefunden, daß Verbindungen der Formel (I) aus den Enantiomeren-Gemischen durch enzymatische Esterspaltung (Hydrolyse oder Alkoholyse) in optisch reiner Form erhalten werden können.
Gegenstand der vorliegenden Erfindung ist somit ein Verfahren zur kinetischen Racematspaltung von Verbindungen der Formel (I), 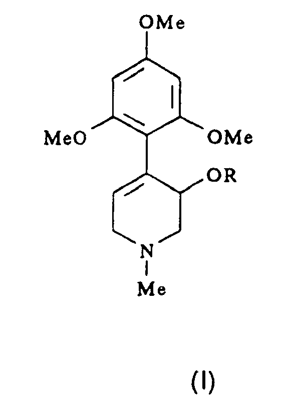 das dadurch gekennzeichnet ist, daß man Enantiomeren-Gemische bzw. racemische Gemische von Verbindungen der Formel (I), in der
- R: steht für COR¹ mit R¹ = (C₁-C₁₆)-Alkyl, (C₂-C₁₆)-Alkenyl bzw. (C₃-C₁₆)-Alkinyl, CₙH₂ₙ-Cycloalkyl mit n = 1-16, die verzweigt oder nichtverzweigt sein können und die substituiert sein können mit 1-3 Substituenten aus der Gruppe F, Cl, Br, J, CF₃, CN, NO₂, Hydroxy, Methoxy, Ethoxy und COOR², mit R² = (C₁- C₄)-Alkyl und (C₂-C₄)-Alkenyl, die verzweigt oder nichtverzweigt sein können und die substituiert sein können mit 1-3 Substituenten aus der Gruppe F, Cl, Br, CF₃,
in homogenen oder heterogenen, wäßrigen, wäßrig-organischen oder organischen Medien in Gegenwart eines Enzyms, z. B. einer Lipase oder Esterase, z. B. aus Säugetier-Lebern oder -Bauchspeicheldrüsen oder mikrobiellen Ursprungs, wie beispielsweise aus Candida, Pseudomonas und Aspergillus, oder einer Protease, z. B. aus Bacillus, einer stereoselektiven Hydrolyse oder Alkoholyse bei einer Temperatur von 10 - 80 °C gegebenenfalls in Gegenwart von Cosolventien und eines Puffers unterwirft wobei die Reaktionsmischung vorzugsweise 2 - 50 Gew.-% Ester enthält
und nach Ablauf der Reaktion den nicht umgesetzten Ester (Verbindung der Formel (I) mit R = COR¹) und den gebildeten Alkohol (Verbindung der Formel (I) mit R = H) - und somit beide Enantiomere - trennt.

Das erfindungsgemäße Verfahren ist ökonomisch, einfach und schnell. Die Reaktion erfordert keine äquimolaren Mengen optisch reiner Hilfsstoffe, keine teuren Reagenzien, keine unverhältnismäßig großen Lösungsmittelmengen und keine kostenintensiven Arbeitsschritte. Nach Beendigung der Reaktion kann die Trennung der Produkte bzw. der Enantiomeren durch einfache Maßnahmen, z. B. durch Extraktion, erfolgen.

Bevorzugt steht in den Verbindungen der Formel (I)
- R: für COR¹ mit R¹ = (C₁-C₁₂)-Alkyl, (C₂-C₁₂)-Alkenyl bzw. (C₃-C₁₂)-Alkinyl, CₙH₂ₙ- Cycloalkyl mit n = 1-12, die verzweigt oder nichtverzweigt sein können und die substituiert sein können mit 1-3 Substituenten aus der Gruppe F, Cl, Br, CF₃, CN, NO₂, Hydroxy, Methoxy, Ethoxy und COOR², mit R² = Methyl, Ethyl und Vinyl, die substituiert sein können mit 1-3 Substituenten aus der Gruppe F, Cl, CF₃.

Besonders bevorzugt steht in den Verbindungen der Formel (I)
- R: für COR¹ mit R¹ = (C₁-C₁₀)-Alkyl, (C₂-C₁₀)-Alkenyl bzw. (C₃-C₁₀)-Alkinyl, CₙH₂ₙ- Cycloalkyl mit n = 1-10, die verzweigt oder nichtverzweigt sein können und die substituiert sein können mit 1-3 Substituenten aus der Gruppe F, Cl, Br, CF₃, CN, NO₂, Methoxy, und COOR², mit R² = Methyl, Ethyl und Vinyl, die substituiert sein können mit 1-3 Substituenten aus der Gruppe F, Cl, CF₃.

Ganz besonders bevorzugt steht in den Verbindungen der Formel (I)
- R: für COR¹ mit R¹ = (C₁-C₁₀)-Alkyl, (C₂-C₁₀)-Alkenyl bzw. (C₃-C₁₀)-Alkinyl,
die verzweigt oder nichtverzweigt sein können und die substituiert sein können mit 1-3 Substituenten aus der Gruppe F, Cl, Br, CF₃, und Methoxy.

Bei dem Verfahren geht man vorzugsweise so vor, daß man einen Ester der Formel (I), beispielsweise R = COR¹ mit R¹ = C₃H₇ oder C₈H₁₇ in einer wasser- oder alkoholhaltigen Lösung mit einer Lipase, Esterase oder Protease versetzt und rührt. Es kann vorteilhaft sein, die genannte Lösung zu puffern, z. B. mit Phosphat- oder TRIS [= Tris-(hydroxymethyl)-methylamin]-Puffer. Der Zusatz kann z. B. 0.01-1.0 molar sein. Ein günstiger Pufferbereich ist pH 5-9.

Es kann weiterhin vorteilhaft sein, Cosolventien zuzugeben. Als Cosolvens eignen sich z. B. Dimethoxyethan, Aceton, THF, Dioxan, Hexan, tert.-Butylmethylether und tert.-Butanol. Der Anteil an Cosolvens in der Lösung liegt vorzugsweise bei 10-80 %.

Als Enzyme werden bevorzugt Lipasen und Esterasen , wie z. B. CholesterolEsterase (EC 3.1.1.13) aus Rinderpankreas (Sigma Chemical Co.), Schweineleber-Esterase (PLE (porcine liver esterase), Sigma Chemical Co.), Pankreatin (Fluka und Sigma Chemical Co.), Pankreas-Acetonpulver vom Rind (Sigma Chemical Co.), Leber-Acetonpulver vom Pferd (Sigma Chemical Co.) und Lipase aus Schweinepankreas (PPL (porcine pancreas lipase), Sigma Chemical Co.), Lipase OF aus Candida rugosa (Meito Sangyo) und Lipase AP-6 aus Aspergillus niger (Amano Pharmaceuticals) eingesetzt.

Jedes der genannten Enzyme kann in freier oder in immobilisierter Form (Immobilized Biocatalysts, W. Hartmeier, Springer Verlag Berlin, 1988) eingesetzt werden. Die Enzymmenge wird in Abhängigkeit von der Reaktionsgeschwindigkeit bzw. von der angestrebten Reaktionszeit und von der Art des Enzyms (z. B. frei oder immobilisiert) frei gewählt und ist durch einfache Vorversuche leicht zu bestimmen.

Die Reaktionsmischung enthält vorzugsweise 2-50 Gew.-% Ester, besonders bevorzugt 5-20 %. Die Reaktionstemperatur beträgt 10-80 °C, bevorzugt 20-60 °C, besonders bevorzugt 20-40 °C.

Die Herstellung der Ester (Verbindungen der Formel I mit R = COR¹) erfolgt zweckmäßigerweise aus dem Alkohol (Verbindung der Formel I mit R = H) nach bekannten Methoden der Veresterung (Haslam, Tetrahedron 1980, 36, 2409; Höfle, Steglich, Vorbrüggen, Angew. Chem. 1978, 90, 602) oder wie in der Patentanmeldung HMR 98/L 001 ("Verfahren zur Herstellung von (-)cis-3-Hydroxy-1-methyl-4(R)-(2,4,6-trimethoxyphenyl)piperidin") beschrieben.

Die bei dem Verfahren entstehenden bzw. verbleibenden Produkte lassen sich auf einfache Weise trennen, z. B. durch Extraktion oder chromatographische Methoden. Den verbleibenden Ester erhält man z. B durch Verteilen der Reaktionslösung zwischen Wasser und n-Heptan und Einengen der organischen Phase. Der entstandene Alkohol kann dann aus der wäßrigen Phase mit Essigsäureethylester extrahiert werden. Die Wiedergewinnung des Enzyms kann durch Gefriertrocknung erfolgen. Die Abtrennung (und ggf. spätere Wiederverwendung) des Enzyms kann durch Immobilisierung erleichtert werden.

Durch geeignete Reaktionsführung gelingt es immer, zumindest ein Enantiomeres optisch rein zu erhalten. Strebt man optisch reinen Ester an, sollte der Umsatz über (oder gleich) 50 % sein, strebt man optisch reinen Alkohol an, sollte der Umsatz kleiner (oder gleich) 50 % sein. Die Umsatzbestimmung der enzymatischen Hydrolyse oder Alkoholyse erfolgte mit HPLC (RP 18 LiChrosorb®) , die Bestimmung der optischen Reinheit wurde per HPLC (Chiralpak AD) durchgeführt. Die bei dem Verfahren der Racematspaltung entstehenden bzw. verbleibenden Ester lassen sich nach bekannten Methoden der Esterspaltung (S.J. Salomon, E.G. Mata, O.A. Mascaretti, Tetrahedron 1993, 49, 3691-3748) ohne Inversion oder Racemisierung in den korrespondierenden Alkohol überführen. Umgekehrt läßt sich der entstehende Alkohol nach bekannten Methoden der Veresterung (Haslam, Tetrahedron 1980, 36, 2409) ohne Inversion oder Racemisierung in den korrespondierenden Ester überführen.

Die bei dem Verfahren entstehenden bzw. verbleibenden Produkte lassen sich nach bekannten Methoden, z.B. durch metallkatalysierte Umlagerungen (L.E. Overman, Angew. Chem. 1984, 96, 565-573 und bereits zitierte Literatur), racemisieren und erneut in die Racematspaltung einsetzen. Dies erhöht die Ausbeute auf über 50 %. Beispielsweise lassen sich die Verbindungen der Formel (I) mit R = COR¹ direkt und die der Formel (I) mit R = H z.B. nach Überführung in geeignete Derivate, wie sie in L.E. Overman, Angew. Chem. 1994, 96, 565-573 beschrieben sind, racemisieren. Als Metallkatalysatoren können beispielsweise Hg(II)-, Pd(O)- oder Pd(II)-Verbindungen bzw. -Salze verwendet werden.

Durch die nachfolgende Beispiele soll die vorliegende Erfindung näher erläutert werden.

### Beispiele:

Alle isolierten Produkte bzw. Rohproduktgemische wurden durch ¹H-NMR- und Massen-Spektren bzw per HPLC identifiziert.
Die optische Reinheit der Produkte wurde durch HPLC, z. B an Chiralpak AD 250 X 4.6 (Daicel) bestimmt.

### Beispiel 1:

10 mg des Essigsäureesters [Verbindung der Formel I mit R¹ = COR² und R² = COCH₃] wurden in 1 mL Kaliumphosphat-Puffer (0.1 M, pH = 7.0) / Dimethoxyethan (5:1) vorgelegt. 5 mg Pankreatin wurden zugegeben. Es wurde bei 20-25 °C gerührt bis der Umsatz ca. 40 % (HPLC) erreicht hatte. Dann wurde filtriert, zur Trockne eingeengt und die resultierende Mischung per HPLC (Chiralpak AD 250 x 4.6, n-Hexan + EtOH 5 + 1, Fluß 1 mL/min, 25 °C, 220/240 nm) untersucht:
ee des verbliebenen (R)-Essigsäureesters: 63 %; ee des (S)-Alkohols: 85 %.

### Beispiel 2:

10 mg des Buttersäureesters [Verbindung der Formel 1 mit R¹ = COR² und R² = CO(CH₂)₂CH₃] wurden in 1 mL Kaliumphosphat-Puffer (0.1 M, pH = 7.0)/ Dimethoxyethan (5:1) vorgelegt. 5 mg PPL (Lipase aus Schweinepankreas, Sigma Chemical Co.) wurden zugegeben. Es wurde bei 30 °C gerührt bis der Umsatz ca. 48 % (HPLC) erreicht hatte. Dann wurde filtriert, zur Trockne eingeengt und die resultierende Mischung per HPLC (Chiralpak AD 250 x 4.6, n-Hexan + EtOH 6 + 1, Fluß 1 mL/min, 25 °C, 220/240 nm) untersucht:
ee des (R)-Buttersäureesters: 90 %; ee des (S)-Alkohols: 97 %.

### Beispiel 3:

1.0 g (2.86 mmol) des Buttersäureesters [Verbindung der Formel I mit R¹ = COR² und R² = CO(CH₂)₂CH₃] wurden in 8 mL Dimethoxyethan und 40 mL Kaliumphosphat-Puffer (0.1 M, pH = 7.0) vorgelegt. 90 mg Pankreatin wurden zugegeben. Es wurde bei 22-25 °C gerührt bis der Umsatz 50 % überschritten hatte. Dann wurde im Vakuum eingeengt, mit Wasser versetzt und sechsmal mit ca. 50 mL n-Heptan extrahiert. Nach Trocknen (Na₂SO₄) wurde im Vakuum eingeengt. Man erhielt 450 mg (45 %) des (R)-Buttersäureesters; ee (HPLC): ≥ 99 %. Nach Extraktion der verbliebenen Wasserphase mit Essigsäureethylester, Trocknen (Na₂SO₄) und Einengen im Vakuum erhielt man 190 mg (23.8 %) des (S)-Alkohols; ee (HPLC): 97 %.

### Beispiel 4:

10 mg des Buttersäureesters [Verbindung der Formel I mit R¹ = COR² und R² = CO(CH₂)₂CH₃] wurden in 1 mL Kaliumphosphat-Puffer (0.1 M, pH = 7.0)/Dimethoxyethan (5:1) vorgelegt. 5 mg PPL wurden zugegeben. Es wurde bei 30 °C gerührt bis ein Umsatz von ca. 48 % (HPLC) erreicht war. Dann wurde filtriert, zur Trockne eingeengt und die resultierende Mischung per HPLC (Chiralpak AD 250 x 4.6, n-Hexan + EtOH 6 + 1, Fluß 1 mL/min. 25 °C, 220/240 nm) untersucht:
ee des (R)-Buttersäureesters: 90 %; ee des (S)-Alkohols: 97 %.

### Beispiel 5:

10 mg des Buttersäureesters [Verbindung der Formel I mit R¹ = COR² und R² = CO(CH₂)₂CH₃] wurden in 1 mL Kaliumphosphat-Puffer (0.1 M, pH = 7.0)/ Dimethoxyethan (5:1) vorgelegt. 5 mg PLE (Schweinleber-Esterase, Sigma Chemical Co.) wurden zugegeben. Es wurde bei 30 °C gerührt bis ein Umsatz von ca. 47 % (HPLC) erreicht war. Dann wurde filtriert, zur Trockne eingeengt und die resultierende Mischung per HPLC (Chiralpak AD 250 x 4.6, n-Hexan + EtOH 6 + 1, Fluß 1 mL/min, 25 °C, 220/240 nm) untersucht:
ee des (R)-Buttersäureesters: 88 %; ee des (S)-Alkohols: 97 %.

### Beispiel 6:

10 mg des Capronsäureesters [Verbindung der Formel I mit R¹ = COR² und R² = CO(CH₂)₄CH₃] wurden in 1 mL Kaliumphosphat-Puffer (0.1 M, pH = 7.0)/ Dimethoxyethan (5:1) vorgelegt. 5 mg PLE wurden zugegeben. Es wurde bei 30 °C gerührt bis ein Umsatz von ca. 40 % (HPLC) erreicht war. Dann wurde filtriert, zur Trockne eingeengt und die resultierende Mischung per HPLC (Chiralpak AD 250 x 4.6, n-Hexan + EtOH 6 + 1, Fluß 1 mL/min, 25 °C, 220/240 nm) untersucht:
ee des (R)-Capronsäureesters: 66 %; ee des (S)-Alkohols: 96 %.

### Beispiel 7:

10 mg des Capronsäureesters [Verbindung der Formel I mit R¹ = COR² und R² = CO(CH₂)₄CH₃] wurden in 1 mL Kaliumphosphat-Puffer (0.1 M, pH = 7.0)/ Dimethoxyethan (5: 1) vorgelegt. 5 mg Cholesterolesterase aus Rinderpankreas wurden zugegeben. Es wurde bei 30 °C gerührt bis ein Umsatz von ca. 50 % (HPLC) erreicht war. Dann wurde filtriert, zur Trockne eingeengt und die resultierende Mischung per HPLC (Chiralpak AD 250 x 4.6, n-Hexan + EtOH 6 + 1, Fluß 1 mL/min, 25 °C, 220/240 nm) untersucht:
ee des (R)-Capronsäureesters: ≥ 99.8 %; ee des (S)-Alkohols: ≥ 99.8 %.

### Beispiel 8:

10 mg des Caprinsäureesters [Verbindung der Formel I mit R¹ = COR² und R² = CO(CH₂)₈CH₃] wurden in 1 mL Kaliumphosphat-Puffer (0.1 M, pH = 7.0)/ Dimethoxyethan (5:1) vorgelegt. 5 mg PPL wurden zugegeben. Es wurde bei 30 °C gerührt bis ein Umsatz von ca. 10 % (HPLC) erreicht war. Dann wurde filtriert, zur Trockne eingeengt und die resultierende Mischung per HPLC (Chiralpak AD 250 x 4.6, n-Hexan + EtOH 6 + 1, Fluß 1 mL/min, 25 °C, 220/240 nm) untersucht:
ee des (R)-Caprinsäureesters: ≥ 11 %; ee des (S)-Alkohols: 95 %.

### Beispiel 9:

10 mg des Buttersäureesters [Verbindung der Formel I mit R¹ = COR² und R² = CO(CH₂)₂CH₃] wurden in 1 mL Kaliumphosphat-Puffer (0.1 M, pH = 7.0)/ Dimethoxyethan (5:1) vorgelegt. 5 mg Pferdeleber-Acetonpulver wurden zugegeben. Es wurde bei 30 °C gerührt bis ein Umsatz von ca. 46 % (HPLC) erreicht war. Dann wurde filtriert, zur Trockne eingeengt und die resultierende Mischung per HPLC (Chiralpak AD 250 x 4.6, n-Hexan + EtOH 6 + 1, Fluß 1 mL/min, 25 °C, 220/240 nm) untersucht:
ee des (R)-Buttersäureesters: 82 %; ee des (S)-Alkohols: 96 %.

## Patentansprüche

1. Verfahren zur kinetischen Racematspaltung von Verbindungen der Formel (I), 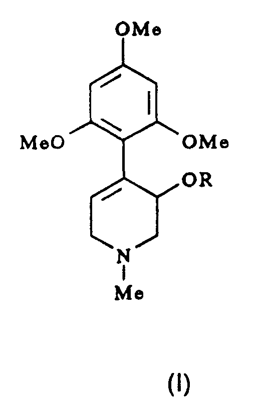 **dadurch gekennzeichnet, daß** man Enantiomeren-Gemische bzw. racemische Gemische von Verbindungen der Formel (I), in der
R steht für COR¹ mit R¹ = (C₁-C₁₆)-Alkyl, (C₂-C₁₆)-Alkenyl bzw. (C₃-C₁₆)-Alkinyl, CₙH₂ₙ-Cycloalkyl mit n = 1-16, die verzweigt oder nichtverzweigt sein können und die substituiert sein können mit 1-3 Substituenten aus der Gruppe F, Cl, Br, J, CF₃, CN, NO₂, Hydroxy, Methoxy, Ethoxy und COOR², mit R² = (C₁- C₄)-Alkyl und (C₂-C₄)-Alkenyl, die verzweigt oder nichtverzweigt sein können und die substituiert sein können mit 1-3 Substituenten aus der Gruppe F, Cl, Br, CF₃,
in homogenen oder heterogenen, wäßrigen, wäßrig-organischen oder organischen Medien in Gegenwart eines Enzyms einer stereoselektiven Hydrolyse oder Alkoholyse bei einer Temperatur von 10 - 80 °C gegebenenfalls in Gegenwart von Cosolventien und eines Puffers unterwirft wobei die Reaktionsmischung vorzugsweise 2 - 50 Gew.-% Ester enthält
und nach Ablauf der Reaktion den nicht umgesetzten Ester (Verbindung der Formel (I) mit R = COR¹) und den gebildeten Alkohol (Verbindung der Formel (I) mit R = H) - und somit beide Enantiomere - trennt.

2. Verfahren zur kinetischen Racematspaltung von Verbindungen der Formel (I), gemäß Anspruch 1, **dadurch gekennzeichnet, daß**
R für COR¹ mit R¹ = (C₁-C₁₂)-Alkyl, (C₂-C₁₂)-Alkenyl bzw. (C₃-C₁₂)-Alkinyl, CₙH₂ₙ- Cycloalkyl mit n = 1-12, die verzweigt oder nichtverzweigt sein können und die substituiert sein können mit 1-3 Substituenten aus der Gruppe F, Cl, Br, CF₃, CN, NO₂, Hydroxy, Methoxy, Ethoxy und COOR², mit R² = Methyl, Ethyl und Vinyl, die substituiert sein können mit 1-3 Substituenten aus der Gruppe F, Cl, CF₃ steht.

3. Verfahren zur kinetischen Racematspaltung von Verbindungen der Formel (I), gemäß Anspruch 1 oder 2, **dadurch gekennzeichnet, daß**
R für COR¹ mit R¹ = (C₁-C₁₀)-Alkyl, (C₂-C₁₀)-Alkenyl bzw. (C₃-C₁₀)-Alkinyl, CₙH₂ₙ- Cycloalkyl mit n = 1-10, die verzweigt oder nichtverzweigt sein können und die substituiert sein können mit 1-3 Substituenten aus der Gruppe F, Cl, Br, CF₃, CN, NO₂, Methoxy, und COOR², mit R² = Methyl, Ethyl und Vinyl, die substituiert sein können mit 1-3 Substituenten aus der Gruppe F, Cl, CF₃ steht.

4. Verfahren zur kinetischen Racematspaltung von Verbindungen der Formel (I) gemäß den Ansprüchen 1 bis 3, **dadurch gekennzeichnet, daß** R für COR¹ mit R¹ = (C₁-C₁₀)-Alkyl, (C₂-C₁₀)-Alkenyl bzw. (C₃-C₁₀)-Alkinyl, die verzweigt oder nichtverzweigt sein können und die substituiert sein können mit 1-3 Substituenten aus der Gruppe F, Cl, Br, CF₃, und Methoxy steht.

5. Verfahren zur kinetischen Racematspaltung von Verbindungen der Formel (I) gemäß den Ansprüchen 1 bis 4, **dadurch gekennzeichnet, daß** als Enzym eine Lipase, Esterase oder Protease verwendet wird.

## Claims

1. A process for the kinetic resolution of racemates of compounds of the formula (I), 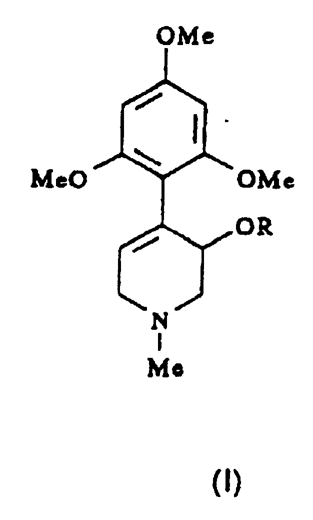 which comprises subjecting enantiomer mixtures or racemic mixtures of compounds of the formula (I), in which
R is COR¹ where R¹ = (C₁-C₁₆)-alkyl, (C₂-C₁₆)-alkenyl or (C₃-C₁₆)-alkynyl, CₙH₂ₙ-cycloalkyl where n = 1- 16, which can be branched or unbranched and which can be substituted by 1-3 substituents from the group F, Cl, Br, I, CF₃, CN, NO₂, hydroxyl, methoxy, ethoxy and COOR², where R² = (C₁-C₄)-alkyl and (C₂-C₄)-alkenyl, which can be branched or unbranched and which can be substituted by 1-3 substituents from the group consisting of F, Cl, Br, CF₃,
in homogeneous or heterogeneous, aqueous, aqueous/organic or organic media in the presence of an enzyme, to a stereoselective hydrolysis or alcoholysis at a temperature of 10-80°C, if appropriate in the presence of cosolvents and of a buffer, the reaction mixture preferably containing 2-50% by weight of ester
and, after the reaction has taken place, separating the unreacted ester (compound of the formula (I) where R = COR¹) and the alcohol formed (compound of the formula (I) where R = H) - and thus the two enantiomers.

2. The process for the kinetic resolution of racemates of compounds of the formula (I), as claimed in claim 1, wherein
R is COR¹ where R¹ = (C₁-C₁₂)-alkyl, (C₂-C₁₂)-alkenyl or (C₃-C₁₂)-alkynyl, CₙH₂ₙ-cycloalkyl where n = 1- 12, which can be branched or unbranched and which can be substituted by 1-3 substituents from the group consisting of F, Cl, Br, CF₃, CN, NO₂, hydroxyl, methoxy, ethoxy and COOR², where R² = methyl, ethyl and vinyl, which can be substituted by 1-3 substituents from the group consisting of F, Cl, CF₃.

3. The process for the kinetic resolution of racemates of compounds of the formula (I), as claimed in claim 1 or 2, wherein
R is COR¹ where R¹ = (C₁-C₁₀)-alkyl, (C₂-C₁₀)-alkenyl or (C₃-C₁₀)-alkynyl, CₙH₂ₙ-cycloalkyl where n = 1- 10, which can be branched or unbranched and which can be substituted by 1-3 substituents from the group consisting of F, Cl, Br, CF₃, CN, NO₂, methoxy, and COOR², where R² = methyl, ethyl and vinyl, which can be substituted by 1-3 substituents from the group consisting of F, Cl, CF₃.

4. The process for the kinetic resolution of racemates of compounds of the formula (I) as claimed in claims 1 to 3, wherein
R is COR¹ where R¹ = (C₁-C₁₀)-alkyl, (C₂-C₁₀)-alkenyl or (C₃-C₁₀)-alkynyl,
which can be branched or unbranched and which can be substituted by 1-3 substituents from the group consisting of F, Cl, Br, CF₃, and methoxy.

5. The process for the kinetic resolution of racemates of compounds of the formula (I) as claimed in claims 1 to 4, wherein the enzyme used is a lipase, esterase or protease.

## Revendications

1. Procédé pour la dissociation cinétique de racémates de composés de formule (I) 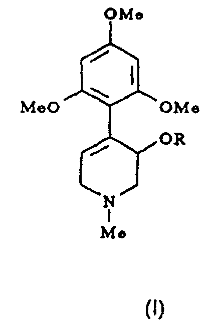 **caractérisé en ce qu'**on soumet des mélanges d'énantiomères ou, selon le cas, des mélanges racémiques de composés de formule (I), dans laquelle R représente COR¹ avec R¹ = (C₁-C₁₆)-alkyle, (C₂-C₁₆)-alcényle ou (C₃-C₁₆)-alcynyle, CₙH₂ₙ-cycloalkyle avec n = 1-16, qui peuvent être ramifiés ou non ramifiés et qui peuvent être substitués par 1-3 substituants du groupe F, Cl, Br, J, CF₃, CN, NO₂, hydroxy, méthoxy, éthoxy et COOR², avec R² = (C₁-C₄)-alkyle et (C₂-C₄)-alcényle, qui peuvent être ramifiés ou non ramifiés et qui peuvent être substitués par 1-3 substituants du groupe F, Cl, Br, CF₃,
dans des milieux homogènes ou hétérogènes, aqueux, aqueux-organiques ou organiques en présence d'une enzyme à une hydrolyse ou une alcoolyse stéréosélective à une température de 10-80°C, le cas échéant en présence de cosolvants et d'un tampon, le mélange contenant de préférence 2-50% en poids d'ester et on sépare après le déroulement de la réaction l'ester non transformé (composé de formule (I) avec R = COR¹) et l'alcool formé (composé de formule (I) avec R = H) - et donc les deux énantiomères.

2. Procédé pour la dissociation cinétique de racémates de composés de formule (I), selon la revendication 1, **caractérisé en ce que**
R représente COR¹ avec R¹ = (C₁-C₁₂)-alkyle, (C₂-C₁₂)-alcényle ou (C₃-C₁₂)-alcynyle, CₙH₂ₙ-cycloalkyle avec n = 1-12, qui peuvent être ramifiés ou non ramifiés et qui peuvent être substitués par 1-3 substituants du groupe F, Cl, Br, CF₃, CN, NO₂, hydroxy, méthoxy, éthoxy et COOR², avec R² = méthyle, éthyle et vinyle, qui peuvent être substitués par 1-3 substituants du groupe F, Cl, CF₃.

3. Procédé pour la dissociation cinétique de racémates de composés de formule (I), selon la revendication 1 ou 2, **caractérisé en ce que**
R représente COR¹ avec R¹ = (C₁-C₁₀)-alkyle, (C₂-C₁₀)-alcényle ou (C₃-C₁₀)-alcynyle, CₙH₂ₙ-cycloalkyle avec n = 1-10, qui peuvent être ramifiés ou non ramifiés et qui peuvent être substitués par 1-3 substituants du groupe F, Cl, Br, CF₃, CN, NO₂, méthoxy et COOR², avec R² = méthyle, éthyle et vinyle, qui peuvent être substitués par 1-3 substituants du groupe F, Cl, CF₃.

4. Procédé pour la dissociation cinétique de racémates de composés de formule (I), selon les revendications 1 à 3, **caractérisé en ce que**
R représente COR¹ avec R¹ = (C₁-C₁₀)-alkyle, (C₂-C₁₀)-alcényle ou (C₃-C₁₀)-alcynyle, qui peuvent être ramifiés ou non ramifiés et qui peuvent être substitués par 1-3 substituants du groupe F, Cl, Br, CF₃ et méthoxy.

5. Procédé pour la dissociation cinétique de racémates de composés de formule (I), selon les revendications 1 à 4, **caractérisé en ce qu'**on utilise comme enzyme une lipase, une estérase ou une protéase.
